# FASCICULE DE BREVET EUROPEEN

(11) **EP 2 623 023 B1**
(45) Date de publication et mention de la délivrance du brevet: **01.10.2014**
(21) Numéro de dépôt: 12192474.0
(22) Date de dépôt: 13.11.2012
(51) Int. Cl.: A61B 5/0205, A61B 5/0452, A61B 5/00, A61B 5/02, A61B 5/0402

(54) **Dispositif médical implantable actif comprenant des moyens de diagnostic de l'insuffisance cardiaque**
Aktives medizinisches Implantat, das mit Diagnosemitteln für Herzinsuffizienz ausgestattet ist
Active implantable medical device including a means for diagnosing heart failure

(30) Priorité: 31.01.2012 FR 1250922
(43) Date de publication de la demande: 07.08.2013
(73) Titulaire: Sorin CRM SAS, 92140 Clamart (FR)
(72) Inventeur: Dumont, Jérôme, 92320 Chatillon (FR); Giorgis, Lionel, 22000 Saint Brieuc (FR)
(74) Mandataire: Dupuis-Latour, Dominique

(56) Documents cités:
- EP-A2- 0 570 896
- WO-A1-2006/105143
- WO-A1-2011/005376
- US-A1- 2011 009 758
- US-B2- 7 570 990

## Description

L'invention concerne les "dispositifs médicaux implantables actifs" tels que définis par la directive 90/385/CEE du 20 juin 1990 du Conseil des Communautés Européennes, plus particulièrement les dispositifs de stimulation cardiaque, resynchronisation et/ou défibrillation destinés aux diagnostics et au traitement des troubles du rythme cardiaque, y compris les implants actifs à visée purement diagnostique.

Elle concerne en particulier le diagnostic préventif du risque de décompensation cardiaque au moyen d'algorithmes d'analyse de signaux recueillis par le dispositif implanté, diagnostic qui peut être particulièrement utile avec les implants mettant en oeuvre des fonctions de resynchronisation.

Ces signaux peuvent être de nature très variée, qu'il s'agisse de signaux délivrés par des électrodes de recueil de l'onde de dépolarisation du myocarde, de mesures reflétant des paramètres hémodynamiques, ou encore de signaux issus de capteurs physiques donnant une indication du niveau d'activité du patient.

Il a été proposé divers algorithmes d'analyse de ces signaux, le plus souvent avec recoupement entre les informations issues de différents capteurs.

Ainsi, les EP 1 767 244 A1 et EP 1 790 382 A1 (ELA Medical) décrivent une technique consistant à analyser parallèlement les signaux délivrés par un capteur d'activité (capteur G, accélérométrique) et un capteur physiologique (capteur MV, de ventilation-minute), le cas échéant avec analyse dissociée activité/repos, de manière à créer un certain nombre d'index spécifiques susceptibles de révéler une aggravation de l'état clinique du patient. Si besoin, une alerte préventive d'insuffisance cardiaque est déclenché lorsqu'un certain nombre de ces index répondent à une batterie de critères prédéfinis.

Le EP 1 867 360 A2 (ELA Medical) propose, outre l'analyse de l'état clinique du patient à partir d'informations issues de capteurs MV et G, d'évaluer également la contractilité cardiaque à partir d'un signal d'accélération endocardiaque (EA) ou de bioimpédance cardiaque. Les algorithmes d'analyse respectifs produisent chacun un signal d'alerte de décompensation cardiaque, et des moyens d'analyse croisée délivrent un signal composite d'alerte préventive, sur différents niveaux, en fonction de l'apparition des signaux d'alarme produits par ces deux algorithmes.

Les WO 2011/005376 A1 et US 2011/0166472 A1 décrivent encore d'autres techniques de diagnostic préventif de décompensation cardiaque, basées sur l'évolution de tendances à long et moyen terme de fréquences respiratoires et d'impédances thoraciques, respectivement.

Tous ces dispositifs peuvent être utilisés notamment pour des techniques de suivi à distance ou *remote monitoring,* où le patient se connecte quotidiennement par une liaison sans fil à un dispositif relié à un site distant, qui transmet les signaux recueillis et produits par l'implant depuis la dernière connexion.

Concrètement, les algorithmes de prévention de décompensation cardiaque utilisés jusqu'à présent génèrent un nombre assez élevé de fausses alertes, généralement des faux positifs, qui sont sans intérêt pour le médecin mais qui risquent d'inquiéter inutilement le patient.

En effet, le paramétrage des différents critères de déclenchement de l'alerte est généralement choisi de manière à privilégier la sensibilité de déclenchement de l'alerte, par ajustement de divers paramètres fixes (seuils), incrémentaux (pourcentages d'augmentation minima ou maxima), ou encore de métarègles analysant l'évolution des index et de leur combinaison sur plusieurs jours.

En effet, la sensibilité et la spécificité (c'est-à-dire la sélectivité de l'analyse) sont généralement considérées comme deux notions antagonistes, dans la mesure où avec les algorithmes connus une augmentation de sensibilité s'accompagne généralement d'une moindre spécificité - avec corrélativement une augmentation des risques de fausses alertes. Inversement, si l'on rend les critères d'alerte plus stricts, on réduit les cas de faux positifs, mais avec le risque de ne pas déclencher d'alerte dans des cas critiques.

Les conséquences des fausses alertes risquent d'être encore plus importantes lorsque le dispositif non seulement délivre une alerte (fonction de diagnostic), mais en outre modifie son fonctionnement pour s'adapter à l'amélioration ou à l'aggravation supposée de la situation du patient, notamment par reprogrammation de certaines de ses fonctions.

Le EP 0 966 987 A1 (ELA Medical) décrit un tel dispositif auto-adaptable en fonction de l'évolution de la situation du patient. On conçoit aisément dans ce cas les conséquences potentiellement graves des fausses détections, qu'il s'agisse aussi bien de faux positifs que de faux négatifs.

Le EP 0 570 896 A2 décrit une technique consistant à calculer une différence entre un épisode de référence et des vecteurs représentatifs de *clusters* de paramètres de cycles-modèles, pour produire un indice d'état clinique du patient et générer le cas échéant une alerte préventive de décompensation cardiaque.

Le US 7 570 990 B prévoit de donner une première indication de décompensation cardiaque sur des cycles qui sont alors marqués "en suspicion", puis dans une seconde passe d'appliquer un algorithme de vérification sur les seuls cycles ainsi marqués.

D'autres techniques encore mettant en oeuvre des cycles-modèles sont décrites par les WO2006/105143 A1 et WO2011/005376 A1.

Le but de l'invention est de mettre à disposition une technique de diagnostic préventif de la décompensation cardiaque qui augmente la spécificité des algorithmes de détection (c'est-à-dire génère beaucoup moins de fausses alertes), mais sans altérer leur sensibilité.

Le but de l'invention est également de mettre à disposition un tel algorithme qui pour sa mise en oeuvre soit peu exigeant en termes de ressources matérielles (processeur, mémoire) et logicielles (complexité, temps de calcul), de manière à pouvoir être utilisé au sein d'un implant, avec les moyens matériels et logiciels actuellement disponibles et sans incidence notable sur le bilan énergétique, notamment sur l'activité du processeur, qui a une incidence directe sur la consommation électrique et par voie de conséquence sur la durée de vie de l'implant.

Le point de départ de l'invention réside dans la constatation de ce que l'information contenue dans les signaux EGM, dans les bruits du coeur (traduits par l'accélération endocardiaque EA) ou dans les épisodes d'effort/récupération n'est pas suffisamment exploitée pour le diagnostic préventif de la décompensation cardiaque, principalement du fait que l'analyse de cette information requiert des ressources de calcul et de mémoire relativement importantes.

L'idée de base de l'invention consiste à mettre en oeuvre des algorithmes connus, relativement simples et peu exigeants en termes de ressources de calcul, pour opérer une discrimination entre une situation qui sera qualifiée de situation "en-suspicion" (réponse positive de l'algorithme de diagnostic) et une situation "hors-suspicion" (dans le cas contraire), mais sans déclenchement d'alerte à ce stade, car le résultat serait trop peu fiable.

Le marqueur "en-suspicion" ou "hors-suspicion" est seulement utilisé pour qualifier et mémoriser un *template* ou "cycle-modèle" constitué d'au moins une information de nature relativement complexe du type évoqué ci-dessus, par exemple un battement cardiaque EGM ou EA complet, éventuellement moyenné, donc une forme d'onde complète, mémorisée en tant que telle.

Le dispositif mémorise (ou met à jour) un tel cycle-modèle "en-suspicion" ainsi qu'un cycle-modèle "hors-suspicion". Le dispositif compare ensuite ces deux cycles-modèles de manière à caractériser leurs différences et en dériver un ou plusieurs indicateurs représentatifs de ces différences. C'est à partir de cet(ces) indicateur(s), reflétant la différence entre les deux cycles-modèles en-suspicion/hors-suspicion que l'alerte préventive de décompensation cardiaque sera déclenchée (ou non).

Concrètement, la spécificité de cette alerte s'avère bien meilleure que celle qui pouvait être obtenue avec les techniques mises en oeuvre jusqu'à présent.

Par ailleurs, on notera que l'analyse opérée consiste seulement à comparer deux cycles-modèles qui ont été mémorisés, ce qui en soi peut être fait de façon relativement simple et efficace, et surtout sans avoir à analyser en continu la donnée d'origine (EGM, EA ...) ayant servi à constituer le cycle-modèle, analyse qui aurait nécessité des ressources de calcul beaucoup plus importantes.

En particulier, la consommation d'espace mémoire sera très réduite, car il suffira d'enregistrer, pour comparaison ultérieure :
- un battement EGM "hors-suspicion" et un battement EGM "en-suspicion", et/ou
- un battement EA "hors-suspicion" et un battement EA "en-suspicion", et/ou
- des paramètres recueillis lors d'un épisode d'effort/récupération "hors-suspicion" et lors d'un épisode comparable d'effort/récupération "en-suspicion", par exemple l'enregistrement d'un cycle décrivant la ventilation-minute en fonction du rythme cardiaque dans chacune de ces deux situations.

Un autre avantage encore de l'invention est la possibilité d'utiliser des indicateurs morphologiques pour comparer les cycles-modèles, par exemple des mesures d'intercorrélation, qui en ce qui concerne le diagnostic de l'insuffisance cardiaque sont des indicateurs beaucoup plus puissants que les indicateurs standard (indicateurs résultant simplement de la comparaison de données ponctuelles telles que amplitude de l'onde R, niveau du pic d'accélération endocardiaque, etc.). Ceci permet d'augmenter encore la spécificité de l'algorithme de détection.

Plus précisément, l'invention propose un dispositif de type connu, notamment d'après le EP 0 570 896 A2 précité, comprenant : des moyens de recueil de données d'activité cardiaque ; des moyens d'analyse aptes à produire périodiquement au moins un indice d'état clinique du patient ; et des moyens générateurs d'une alerte préventive de décompensation cardiaque en fonction dudit au moins un indice d'état clinique du patient ; des moyens de recueil d'épisodes de référence, ces épisodes comprenant des signaux d'activité électrique du myocarde, des signaux d'activité hémodynamique du myocarde, et/ou des indicateurs reflétant la variation de paramètres physiques, d'activité et/ou hémodynamiques entre des phases d'effort et des phases de récupération ; des moyens discriminateurs, aptes à analyser lesdits épisodes en fonction de critères prédéfinis pour attribuer à chacun de ces épisodes un marqueur en-suspicion ou hors-suspicion de décompensation cardiaque ; des moyens d'élaboration et de mémorisation de cycles-modèles, aptes à produire à partir desdits épisodes au moins deux cycles-modèles à partir d'épisodes recueillis dans des situations respectives en-suspicion et hors-suspicion de décompensation cardiaque telles que déterminées par les moyens discriminateurs.

De façon caractéristique, le dispositif comprend des moyens de caractérisation de la différence entre un cycle-modèle en-suspicion et un cycle-modèle hors-suspicion, pour produire en réponse ledit au moins un indice d'état clinique du patient.

Le cycle-modèle comprend notamment : au moins un battement d'électrogramme endocavitaire enregistré au cours d'un épisode répondant à des conditions prédéterminées de rythme cardiaque et d'activité du patient ; et/ou au moins un battement de signal d'accélération endocardiaque enregistré au cours d'un épisode répondant à des conditions prédéterminées de rythme cardiaque et d'activité du patient ; et/ou au moins un tracé caractéristique ventilation-minute vs. rythme cardiaque enregistré au cours d'un épisode comprenant une phase d'effort suivie d'une phase de récupération du patient.

Le dispositif comprend en outre des moyens de mise à jour des cycles-modèles produits par les moyens d'élaboration et de mémorisation de cycles-modèles, chaque cycle-modèle étant notamment défini par lesdits moyens d'élaboration et de mémorisation de cycles-modèles en tant que combinaison du dernier cycle-modèle produit avec au moins un cycle-modèle produit antérieurement.

Les moyens d'analyse peuvent avantageusement être des moyens aptes à calculer un indice d'état clinique à partir d'une pluralité de cycles-modèles antérieurs moyennés.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre sous forme de schéma par blocs le principe général de l'invention, avec les divers modules fonctionnels concernés.
La Figure 2 est un exemple de cycle-modèle de signal constitué par un battement EGM, dans une situation hors-suspicion et dans une situation en-suspicion.
La Figure 3 est un exemple de cycle-modèle de signal constitué par une onde EA, dans une situation hors-suspicion et dans une situation en-suspicion.
La Figure 4 est un exemple de cycle-modèle de signal constitué par un cycle correspondant au tracé de la ventilation-minute MV en fonction du rythme cardiaque HR lors d'une phase de récupération succédant à une phase d'effort.
La Figure 5 est un organigramme décrivant les actions successives effectuées lorsque de nouvelles données valides peuvent être exploitées pour mettre à jour les cycles-modèles et produire une alerte plus fiable que l'alerte initiale.
La Figure 6 est un algorithme illustrant l'analyse des données d'entrée et leur classification par attribution d'un marqueur en/hors-suspicion.
La Figure 7 est un exemple d'algorithme de détection de décompensation cardiaque, permettant de discriminer les états hors-suspicion et en-suspicion.
Les Figures 8a et 8b illustrent deux techniques possibles de mise à jour des cycles-modèles mémorisés pour permettre la mise en oeuvre de l'invention.
La Figure 9 illustre de façon schématique la manière dont sont sélectionnés les battements EGM ou EA qui seront utilisés pour créer le cycle-modèle moyenné selon l'une ou l'autre des techniques représentées Figures 8a et 8b.
La Figure 10 illustre deux exemples d'indicateurs permettant de qualifier la différence en/hors suspicion entre un cycle-modèle correspondant au tracé de la ventilation-minute MV en fonction du rythme cardiaque HR lors d'une phase de récupération succédant à une phase d'effort.
La Figure 11 est un exemple d'exécution temporelle de l'invention, montrant la succession des différents marqueurs et indices au cours du temps, sur une période de plusieurs jours.

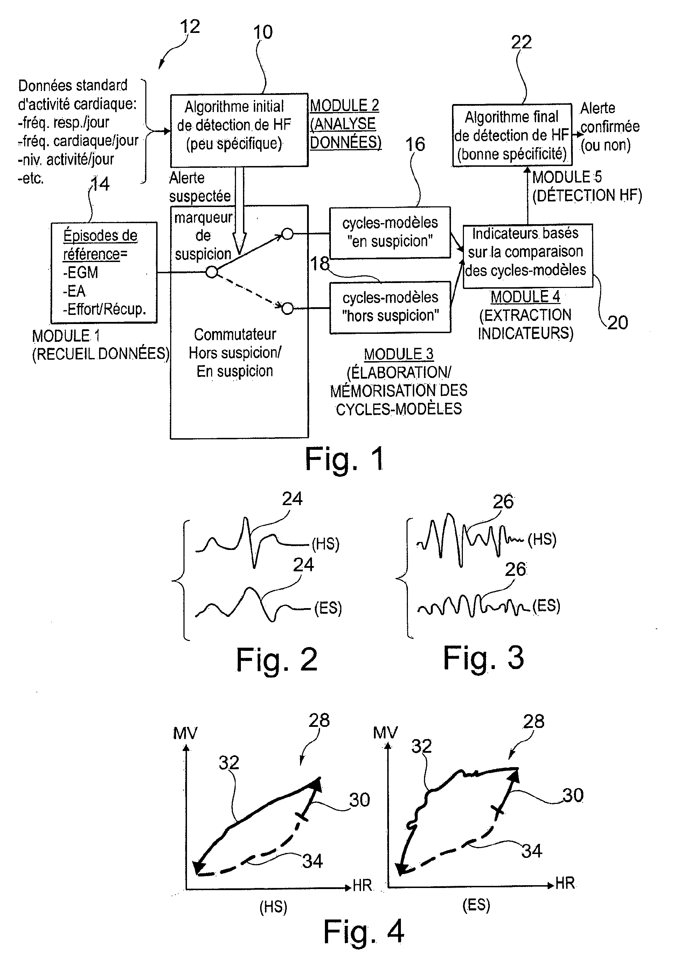

La Figure 1 illustre sous forme de schéma par blocs le principe général de l'invention, avec l'imbrication des différents modules de traitement permettant de conduire à la délivrance (ou non) d'une alerte préventive de décompensation cardiaque (HF, *Heart Failure*).

Un algorithme 10 d'un type en lui-même connu et que l'on qualifiera d"'algorithme initial" analyse des données standard d'activité cardiaque 12 telles que niveau d'activité, rythme respiratoire, fréquence cardiaque, etc., enregistrées au jour le jour.

Dans la technique antérieure, cet algorithme 10 est utilisé pour délivrer, ou non, une alerte de décompensation cardiaque (HF). En revanche, dans l'invention, le résultat de l'analyse ne sera pas un indicateur d'alerte, mais simplement un marqueur intermédiaire permettant de discriminer entre une situation qui sera considérée comme "en suspicion de décompensation cardiaque" (ES) ou, dans le cas contraire, "hors suspicion de décompensation cardiaque" (HS). L'attribution de ce marqueur HS ou ES est schématisé par le bloc "Commutateur Hors suspicion/En suspicion".

Un certain nombre de données, en elles-mêmes relativement complexes et riches en information, sont par ailleurs recueillies par le dispositif (bloc 14). Ces données peuvent être notamment :
- des signaux d'activité électrique du myocarde, notamment un électrogramme endocavitaire (EGM) ;
- des signaux d'activité hémodynamique, tels qu'un signal d'accélération endocardiaque (EA) ou de bioimpédance cardiaque ;
- des signaux reflétant les variations de divers paramètres (fréquence cardiaque, fréquence et amplitude ventilatoires, activité, etc.) lors d'une alternance de phase d'effort et de récupération.

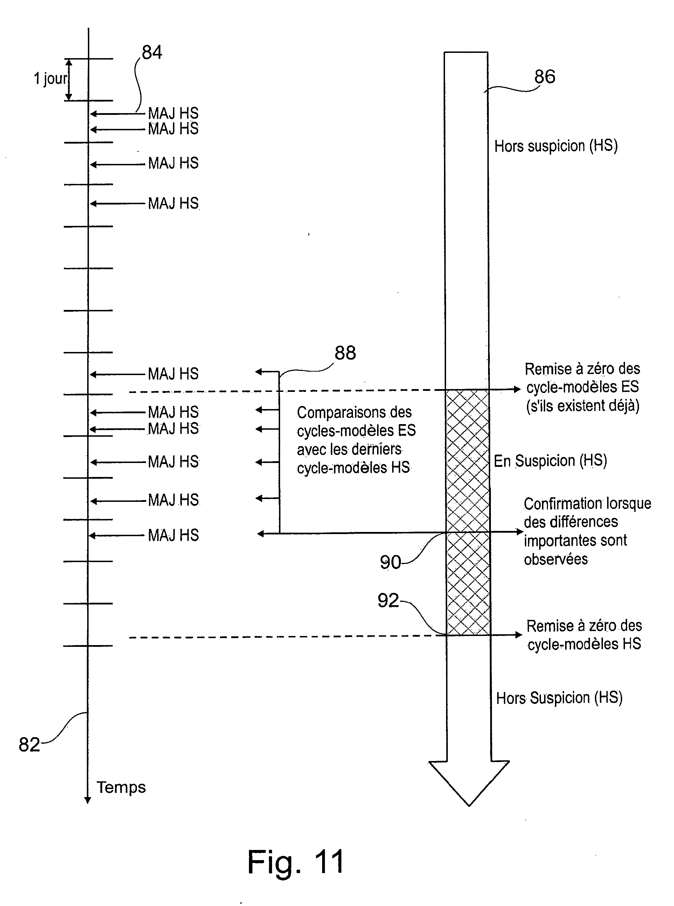

Ces données vont être mémorisées sous forme d'un "cycle-modèle", éventuellement après moyennage journalier ou autre traitement préalable de mise à jour, et mémorisées sous forme soit d'un cycle-modèle "en-suspicion" (bloc 16), soit d'un cycle-modèle "hors-suspicion" (bloc 18) selon la situation du marqueur de suspicion (commutateur "Hors suspicion/En suspicion") au moment où les données d'entrée sont recueillies. Les deux cycles-modèles mémorisés 16 et 18 seront ultérieurement comparés (bloc 20) de manière à caractériser la différence entre ces deux cycles-modèles (en-suspicion vs. hors-suspicion). Cette comparaison sera avantageusement effectuée, comme on le décrira plus bas en référence à la Figure 11, lorsque le cycle-modèle "en-suspicion" est mis à jour, car c'est à ce moment là que peut, potentiellement, être déclenchée une alerte.

Un ou plusieurs indices d'état clinique du patient sont produits en fonction de critères prédéterminés, et ce ou ces indices sont ensuite analysés (bloc 22) pour détecter une situation de décompensation cardiaque potentielle (bloc 22) et délivrer (ou non) une alerte correspondante.

La Figure 2 donne un premier exemple de données d'entrée recueillies par le bloc 14, en l'espèce un signal EGM recueilli sur la durée d'un battement cardiaque 24, par exemple un signal unipolaire enregistré dans des conditions prédéterminées : au repos, pendant la nuit, à fréquence cardiaque constante, etc. Le dispositif enregistre un tel cycle-modèle 24 dans une situation hors-suspicion et un autre cycle-modèle 24 dans une situation en-suspicion.

La Figure 3 donne un deuxième exemple de données d'entrée recueillies par le bloc 14, en l'espèce un signal EA recueilli dans des conditions prédéterminées sur la durée d'un battement cardiaque 26. Le dispositif enregistre un tel cycle-modèle 26 dans une situation hors-suspicion et un autre cycle-modèle 26 dans une situation en-suspicion.

La Figure 4 donne un troisième exemple de données d'entrée recueillies par le bloc 14, en l'espèce un cycle 28 donnant la variation de la ventilation-minute MV en fonction de la fréquence cardiaque HR. Ce cycle est enregistré lors d'une phase d'effort suivie d'une phase de récupération. La boucle décrite comprend ainsi une partie 32 correspondant au démarrage de la phase d'effort, suivie d'une partie 32 correspondant à la récupération après l'arrêt de cet effort. La boucle comprend également une partie 34 non enregistrée, pour laquelle les indicateurs de déclenchement d'enregistrement (durée minimale d'effort, niveau minimal d'activité, de ventilation, etc.) ne sont pas encore atteints, à la différence des parties 30 et 32 qui, en revanche, correspondent à une situation où ces indicateurs de déclenchement ont été atteints, confirmant qu'il y a bien lieu d'enregistrer le cycle effort/récupération.

On va maintenant décrire plus en détail la manière dont sont exécutées par l'algorithme de l'invention les différentes étapes que l'on vient d'évoquer.

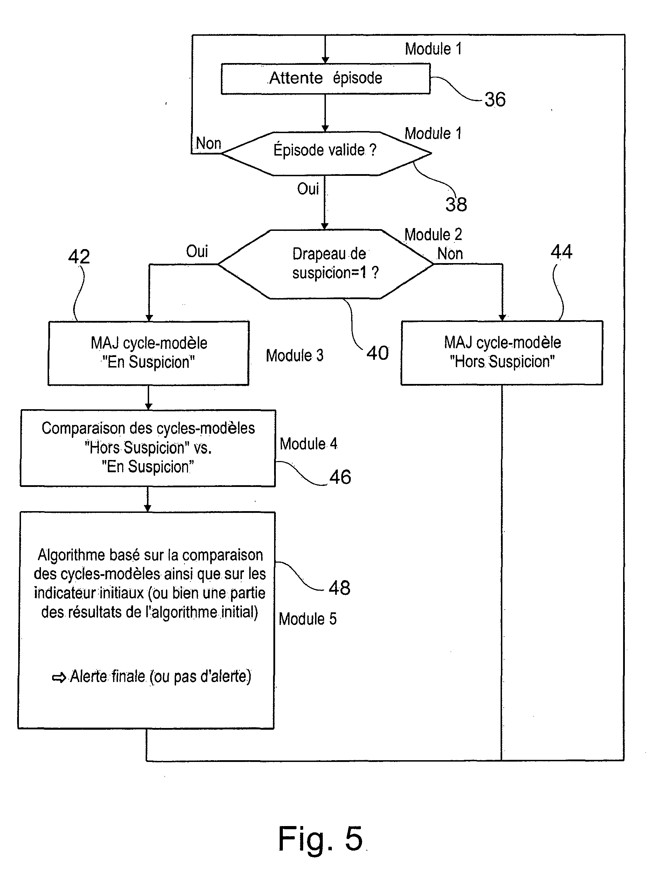

La Figure 5 illustre une chronologie générale du procédé mis en oeuvre par l'invention.

La première phase (bloc 36), exécutée par le Module n°1 de recueil des données (bloc 14 sur la Figure 1), consiste à attendre un épisode qui sera reconnu comme valide, c'est-à-dire correspondant à un certain nombre de conditions prédéterminées : fréquence cardiaque stable, état de repos du patient, absence de trouble du rythme, etc. Si l'épisode est considéré comme valide (bloc 38), une classification est opérée en fonction du marqueur de suspicion en/hors-suspicion (bloc 40) délivré par le Module n°2 d'analyse des données (bloc 10 sur la Figure 1) par application de l'algorithme initial, connu mais peu spécifique.

Le cycle-modèle correspondant "en-suspicion" (bloc 42) ou "hors-suspicion" (bloc 44) est mémorisé (ou mis à jour), cette étape correspondant au Module n° 3 de la Figure 1 (blocs 16, 18).

L'étape suivante (bloc 46) consiste à comparer les deux cycles-modèles hors-suspicion et en-suspicion de manière à caractériser leurs différences et extraire ainsi un ou plusieurs indicateurs basés sur cette comparaison (Module n°4, bloc 20 de la Figure 1).

Ces différents indicateurs sont ensuite traités (bloc 48) par un second algorithme de diagnostic de décompensation cardiaque, de manière à délivrer le cas échéant une alerte finale, spécifique, de risque de décompensation cardiaque à court terme (Module n°5, bloc 22 de la Figure 1).

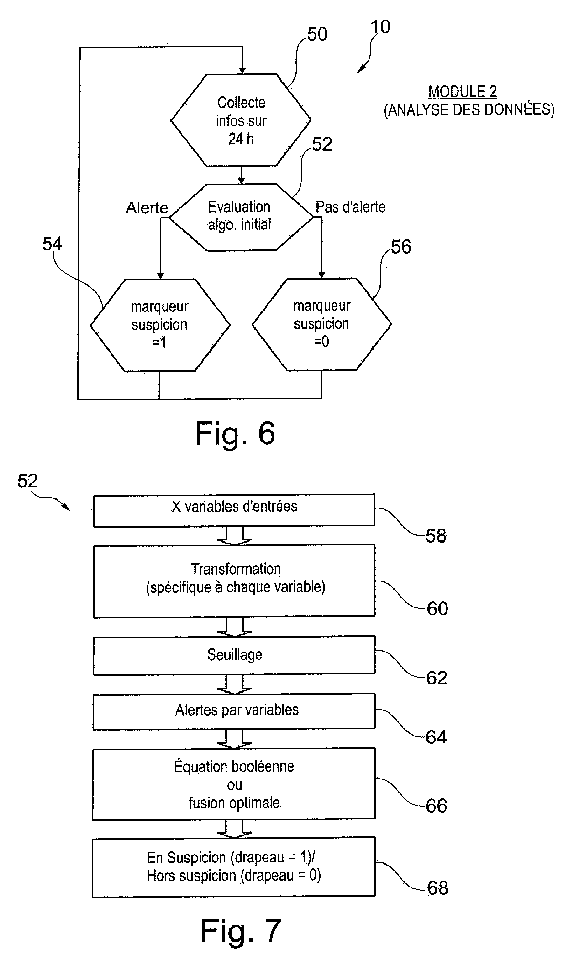

La Figure 6 illustre les étapes du Module n°2 ayant pour objet d'analyser les données standard d'activité cardiaque 12 enregistrées au jour le jour, afin d'établir le caractère "hors-suspicion" ou "en-suspicion" de la période au cours de laquelle sont recueillies les données destinées à constituer les cycles-modèles.

Les données en question (niveau d'activité, rythme respiratoire, fréquence cardiaque, etc.) sont recueillies par exemple sur une période de 24 heures (bloc 50), puis sont évaluées (bloc 52) par l'algorithme initial, d'un type en lui-même connu. Si le résultat de cette analyse est du type "déclenchement d'une alerte", alors on se contentera de positionner un marqueur "en-suspicion" (bloc 54) ; dans le cas contraire ce marqueur sera positionné à "hors-suspicion" (bloc 56).

La Figure 7 illustre un exemple d'algorithme 52. Cet algorithme peut être n'importe quel algorithme connu générant des alertes de prédiction de décompensation cardiaque basé notamment sur des données journalières, tel que par exemple l'algorithme décrit dans les EP 1 867 360 A2 ou WO 2011/005373 A précités.

Un certain nombre de variables d'entrée sont recueillies (bloc 58) telles que :
- impédance transthoracique,
- durée de la période respiratoire,
- fréquence cardiaque,
- temps d'activité,
- taux d'extrasystoles ventriculaires, etc.

Ces différentes variables sont transformées et pondérées (bloc 60), avec une sensibilité et une spécificité propres à chacune de ces variables ou à une de leurs combinaisons (par exemple le nombre de cycles respiratoires pour lesquels l'impédance mesurée est au-dessous d'un seuil donné, etc.).

Des seuils sont appliqués (bloc 62), conduisant à des alertes propres à chaque variable (bloc 64). Ces alertes sont ensuite combinées entre elles (bloc 66) pour produire une alerte globale qui, dans le cas de la présente invention, sera considérée comme un marqueur de situation en-suspicion (bloc 68) ou, dans le cas contraire, comme un marqueur de situation hors-suspicion.

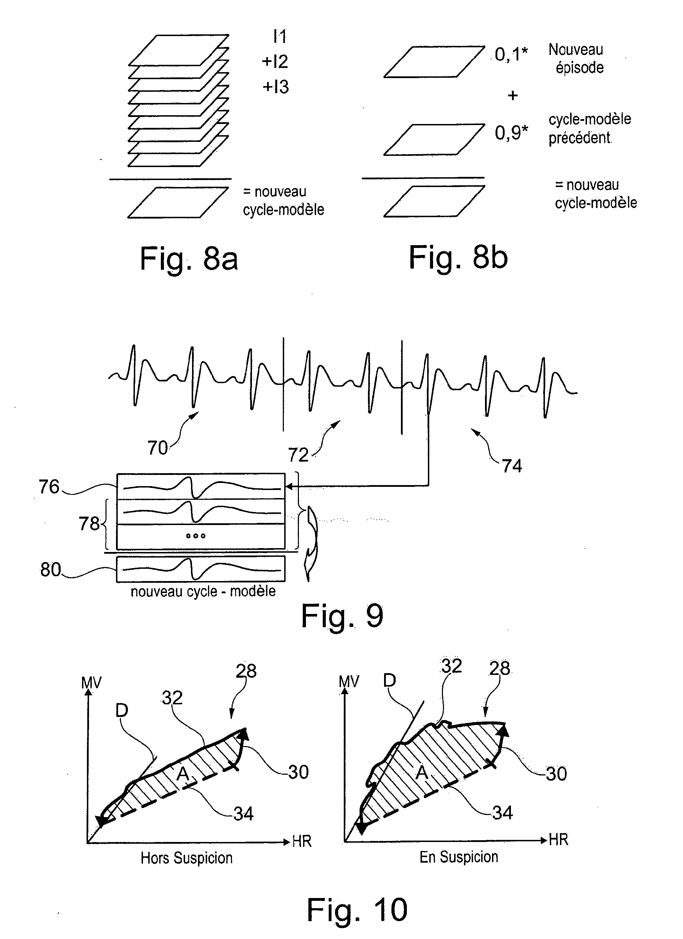

Les Figures 8a et 8b illustrent deux exemples de gestion de la mise à jour des cycles-modèles (en-suspicion ou bien hors-suspicion) exécutée par le Module n° 3 de la Figure 1.

On peut utiliser par exemple, comme illustré Figure 8a, une technique de "mise à jour avec oubli" qui nécessite de conserver *n* enregistrements temporaires de la donnée, le nouvel enregistrement recueilli remplaçant le plus ancien enregistrement temporaire et le cycle-modèle finalement mémorisé étant la moyenne des *n* enregistrements temporaires. Si l'on recueille un épisode bruité, l'incidence de celui-ci sera réduite du fait du moyennage, et à la longue il disparaîtra des enregistrements mémorisés et n'affectera plus le cycle-modèle moyen.

La Figure 8b illustre une autre technique, de type "enregistrement exponentiel", qui nécessite seulement l'enregistrement de la dernière donnée recueillie, en lui donnant une pondération (par exemple de 10 %) par rapport à la moyenne des *n* enregistrements précédemment recueillis et moyennés, affectée quant à elle d'une pondération de 90 %. Un bruit important ou une erreur de mesure auront une répercussion sur le cycle-modèle utile pendant un délai plus long, mais en revanche la quantité de mémoire nécessaire sera beaucoup plus faible que dans le cas précédent.

Les données qui servent à constituer les cycles-modèles moyennés doivent être aussi fiables que possible, et enregistrées dans des conditions comparables ; ceci sous-entend qu'il faudra vérifier l'absence de bruits parasites et que les conditions lors de l'enregistrement de ces données seront constantes dans le temps.

Pour détecter un risque de décompensation cardiaque, il faut analyser les données en période "hors-suspicion" sur une durée assez longue, par exemple établir un cycle-modèle qui représente les dix derniers jours, avec un cycle par jour. Il faut en effet s'assurer que ce cycle-modèle représente bien un état "hors décompensation". Or, si une décompensation commence à apparaître, elle risque de "polluer" les quelques derniers jours de cette période (le temps que l'algorithme initial commence à avérer la suspicion). En revanche, pour un cycle-modèle en suspicion une résolution plus courte, typiquement de 3 à 6 heures, est nécessaire pour disposer en un minimum de temps d'un nombre de cycles enregistrés suffisant pour fournir rapidement un cycle-modèle "en-suspicion", afin de pouvoir faire rapidement la comparaison avec le cycle modèle "hors-suspicion" déjà mémorisé et moyenné. Cette résolution de 3 à 6 heures est un compromis entre une résolution plus courte qui impliquerait un temps de calcul élevé, et inversement une résolution trop faible qui limiterait la précocité de la détection du risque de décompensation. Toutefois, si l'on souhaite que les mises à jour des cycles-modèles soient réalisées dans des conditions vraiment identiques, on pourra éventuellement attendre une période de sommeil (pour les données constituées d'un battement EGM ou EA).

Si la donnée d'entrée est un cycle effort/récupération et non un battement EGM ou EA, un "effort" sera défini comme un épisode caractérisé par le franchissement de seuils d'activité, de ventilation et/ou de fréquence cardiaque, et de durée minimale, par exemple une durée d'effort supérieure ou égale à 2 minutes. Un épisode d'effort/récupération peut être pris en compte s'il survient par exemple au moins une heure après le précédent épisode validé. Ce laps de temps est plus faible que le temps de latence prévu pour l'enregistrement des battements EGM ou EA (3 heures), car les épisodes d'effort/récupération peuvent être très variables, et si l'on veut un cycle-modèle fiable il faudra sans doute un plus grand nombre d'épisodes.

La Figure 9 illustre de façon schématique la manière dont sont sélectionnés les battements EGM ou EA qui seront utilisés pour créer le cycle-modèle moyenné selon l'une ou l'autre des techniques représentées Figures 8a et 8b.

Sur la Figure 9, on a schématisé en 70, 72, 74 ... une succession de battements, pour lesquels on attendra en tout état de cause l'écoulement d'un délai d'au moins 3 heures depuis le précédent enregistrement d'un battement (si l'on veut un battement en condition nocturne, on attendra une période de 24 heures). Une fois ce délai écoulé, on prendra en compte le premier battement vérifiant par exemple les conditions suivantes :
- fréquence cardiaque comprise entre la fréquence de base et la fréquence de base + 10 bpm (ainsi le battement 72, trop rapide, ne sera pas considéré) ;
- battement correspondant à une dépolarisation spontanée ventriculaire;
- absence d'effort (si les capteurs d'activité sont disponibles) ;
- rapport signal/bruit par exemple supérieur à 40 dB, afin d'éliminer les épisodes trop bruités.

Si ces conditions sont vérifiées, par exemple pour le battement 74, celui-ci est enregistré (comme en 76), éventuellement après recalage temporel par rapport aux battements antérieurement mémorisés (comme en 78). Le nouveau cycle-modèle 80 mis à jour est alors calculé par l'une des méthodes décrites plus haut à propos des figures 8a et 8b, c'est-à-dire un moyennage avec oubli ou un moyennage exponentiel.

L'extraction des indicateurs basés sur la comparaison des deux cycles-modèles en-suspicion et hors-suspicion (opérée par le Module n°4, bloc 20 de la Figure 1) peut être exécutée de diverses manières, selon le type de données constituant le cycle-modèle.

Pour un cycle-modèle constitué d'un battement EGM, on peut extraire les indicateurs suivants :
- différence d'amplitude de l'onde R entre le battement hors-suspicion et le battement en-suspicion,
- différence de la durée du QRS,
- différence d'intervalle PR,
- apparition d'une désynchronisation VV,
- indicateur morphologique permettant de comparer deux battements EGM, tels que : distance euclidienne, *Dynamic Time Warping* (DTW), calcul d'un taux d'intercorrélation, descripteur géométrique tel que la norme du vecteur tangent (ces deux techniques étant par exemple explicitées dans le EP 2 324 885 A1 (SORIN CRM) dans le cadre d'un test de capture).

Lorsque le cycle-modèle est constitué d'une onde EA :
- différence d'amplitude entre le premier pic d'accélération endocardiaque PEA1 et/ou le second pic PEA2,
- indicateur morphologique tel que distance euclidienne après recalage temporel des deux ondes, *Dynamic Time Warping,* intercorrélation, etc.

Dans le cas où le cycle-modèle est une courbe ventilation-minute vs. rythme cardiaque :
- comparaison des aires sous les courbes,
- comparaison des maxima des rapports MV/HR,
- comparaison directe des nombres d'échantillons totaux dans les histogrammes hors-suspicion et en-suspicion, etc.

La Figure 10 illustre en particulier ces paramètres, où l'on voit que dans la situation "en-suspicion" l'aire A est plus importante, ainsi que le maximum de MV/HR, paramètre représenté par la pente de la droite D. L'aire peut en particulier être évaluée très simplement après numérisation des échantillons, par une simple sommation qui peut être exécutée avec des ressources de calcul extrêmement réduites et de façon très rapide.

L'alerte finale est le résultat de l'analyse opérée par l'algorithme de détection du Module n°5.

Cet algorithme pondère les différents indicateurs résultant de la comparaison des deux cycles-modèles en-suspicion et hors-suspicion (indicateurs produits par le Module n° 4), éventuellement et avantageusement combinés à des indicateurs délivrés par ailleurs par l'algorithme de détection initial (celui du Module n° 2).

Les indicateurs résultant de la comparaison des deux cycles-modèles en/hors-suspicion sont par exemple :
- alerte si le taux de corrélation des battements EGM en/hors-suspicion est inférieur à 0,8 ;
- alerte si le taux de corrélation entre les battements EA en/horssuspicion est inférieur à 0,6 ;
- alerte si la différence moyenne entre les deux histogrammes des courbes effort/récupération en/hors-suspicion est supérieure à 10 %, etc.

Les indicateurs donnés par l'algorithme initial sont par exemple :
- alerte si l'impédance mesurée décroît de 200 ohms sur 20 jours;
- alerte si la période respiratoire mesurée décroît de 1,5 seconde sur 10 jours ;
- alerte si le nombre de cycles respiratoires perturbés augmente de 1000 sur 20 jours ;
- alerte si la fréquence cardiaque mesurée augmente de 15 bpm sur 20 jours ;
- alerte si le temps en activité diminue d'au moins 1 heure sur 20 jours;
- alerte si le nombre d'extrasystoles augmente de 1000 sur 20 jours, etc. Ces différentes alertes sont combinées et pondérées chacune par un facteur propre, permettant de délivrer une alerte unique, globale. Les pondérations respectives sont choisies en fonction de la sensibilité et de la spécificité de chaque alerte, évaluées sur une base de données cliniques de référence.

La Figure 11 est un exemple de chronologie des résultats de mise en oeuvre de la technique de l'invention, sur une période de plusieurs jours. L'axe des temps 82 indique, jour après jour, les mises à jour des cycles-modèles, schématisées par les flèches 84, chaque mise à jour correspondant à un nouvel épisode validé.

Ces mises à jour (MAJ) peuvent se situer dans un état hors-suspicion (HS) ou en-suspicion (ES), indiqué sur la ligne temporelle 86.

L'étape de comparaison des cycles-modèles, schématisée par les doubles flèches 88, est exécutée dès que l'on a pu mémoriser au moins un cycle-modèle hors-suspicion et au moins un cycle-modèle en-suspicion. La comparaison est effectuée à chaque mise à jour d'un cycle-modèle "en-suspicion", car c'est à ce moment là que peut, potentiellement, être déclenchée une alerte.

Cette comparaison est réitérée à chaque mise à jour d'un cycle-modèle en-suspicion (nouvel épisode validé). Lorsque les différences entre cycles-modèles hors-suspicion/en-suspicion sont trop importantes, alors une alerte peut être délivrée, comme en 90.

Lorsque la situation revient à un état "hors-suspicion", alors le cycle-modèle hors-suspicion est réinitialisé (comme en 92), jusqu'au prochain épisode valide hors-suspicion, où ce cycle-modèle sera à nouveau mémorisé pour utilisation ultérieure.

## Revendications

1. Un dispositif médical implantable actif, notamment un dispositif de stimulation, resynchronisation et/ou défibrillation, ou un implant à visée diagnostique, comprenant des moyens de diagnostic de l'insuffisance cardiaque, avec :
- des moyens (12) de recueil de données d'activité cardiaque;
- des moyens d'analyse aptes à produire périodiquement au moins un indice d'état clinique du patient ; et
- des moyens (22) générateurs d'une alerte préventive de décompensation cardiaque en fonction dudit au moins un indice d'état clinique du patient ;
- des moyens (14) de recueil d'épisodes de référence, ces épisodes comprenant : des signaux d'activité électrique du myocarde ; des signaux d'activité hémodynamique du myocarde ; et/ou des indicateurs reflétant la variation de paramètres physiques, d'activité et/ou hémodynamiques entre des phases d'effort et des phases de récupération ;
- des moyens discriminateurs (10), aptes à analyser lesdits épisodes en fonction de critères prédéfinis pour attribuer à chacun de ces épisodes un marqueur en-suspicion ou hors-suspicion de décompensation cardiaque ; et
- des moyens (16, 18) d'élaboration et de mémorisation de cycles-modèles, aptes à produire à partir desdits épisodes au moins deux cycles-modèles à partir d'épisodes recueillis dans des situations respectives en-suspicion et hors-suspicion de décompensation cardiaque telles que déterminées par les moyens discriminateurs (10, 12), dispositif **caractérisé en ce qu'il** comprend en outre :
- des moyens (20) de caractérisation de la différence entre un cycle-modèle en-suspicion et un cycle-modèle hors-suspicion, pour produire en réponse ledit au moins un indice d'état clinique du patient.

2. Le dispositif de la revendication 1, dans lequel ledit cycle-modèle comprend au moins un battement d'électrogramme endocavitaire enregistré au cours d'un épisode répondant à des conditions prédéterminées de rythme cardiaque et d'activité du patient.

3. Le dispositif de la revendication 1, dans lequel ledit cycle-modèle comprend au moins un battement de signal d'accélération endocardiaque enregistré au cours d'un épisode répondant à des conditions prédéterminées de rythme cardiaque et d'activité du patient.

4. Le dispositif de la revendication 1, dans lequel ledit cycle-modèle comprend au moins un tracé caractéristique ventilation-minute vs. rythme cardiaque enregistré au cours d'un épisode comprenant une phase d'effort suivie d'une phase de récupération du patient.

5. Le dispositif de la revendication 1, comprenant en outre des moyens de mise à jour des cycles-modèles produits par lesdits moyens d'élaboration et de mémorisation de cycles-modèles.

6. Le dispositif de la revendication 5, dans lequel chaque cycle-modèle défini par lesdits moyens d'élaboration et de mémorisation de cycles-modèles est une combinaison du dernier cycle-modèle produit avec au moins un cycle-modèle produit antérieurement.

7. Le dispositif de la revendication 1, dans lequel les moyens d'analyse sont des moyens aptes à calculer un indice d'état clinique à partir d'une pluralité de cycles-modèles antérieurs moyennés.

## Patentansprüche

1. Implantierbare aktive medizinische Vorrichtung, insbesondere Vorrichtung zur Stimulation, Neusynchronisation und/oder Defibrillation oder Diagnoseimplantat, die bzw. das Mittel zum Diagnostizieren einer Herzinsuffizienz umfasst, mit:
- Mitteln (12) zum Empfangen von Herzaktivitätsdaten;
- Analysemitteln, die periodisch wenigstens eine Angabe des klinischen Zustands des Patienten erzeugen können; und
- Mitteln (22) zum Erzeugen einer präventiven Warnung einer Herzkompensationsstörung als Funktion der wenigstens einen Angabe des klinischen Zustands des Patienten;
- Mitteln (14) zum Empfangen von Referenzepisoden, wobei diese Episoden enthalten: Signale der elektrischen Aktivität des Myokards; Signale der hämodynamischen Aktivität des Myokards; und/oder Indikatoren, die die Veränderung von physischen Parametern, Aktivitätsparametern und/oder hämodynamischen Parametern zwischen Anstrengungsphasen und Rekonvaleszenzphasen wiedergeben;
- Diskriminatormitteln (10), die die Episoden als Funktion von im Voraus definierten Kriterien analysieren können, um jeder dieser Episoden einen Markierer für einen Verdacht oder für keinen Verdacht auf eine Herzkompensationsstörung zuzuweisen; und
- Mitteln (16, 18) zum Entwickeln und Speichern von Modellzyklen, die anhand dieser Episoden wenigstens zwei Modellzyklen ausgehend von gesammelten Episoden in Situationen mit Verdacht bzw. ohne Verdacht auf eine Herzkompensationsstörung wie durch die Diskriminatormittel (10, 12) bestimmt erzeugen können,
wobei die Vorrichtung **dadurch gekennzeichnet ist, dass** sie außerdem Folgendes umfasst:
- Mittel (20) zum Charakterisieren des Unterschieds zwischen einem Modellzyklus mit Verdacht und einem Modellzyklus ohne Verdacht, um als Antwort darauf die wenigstens eine Angabe des klinischen Zustands des Patienten zu erzeugen.

2. Vorrichtung nach Anspruch 1, wobei der Modellzyklus wenigstens einen endokavitären Elektrogrammausschlag enthält, der während einer Episode aufgezeichnet wird, die eine Antwort auf vorgegebene Bedingungen des Herzrhythmus und der Aktivität des Patienten darstellt.

3. Vorrichtung nach Anspruch 1, wobei der Modellzyklus wenigstens einen Signalausschlag der endokardialen Beschleunigung umfasst, der während einer Episode aufgezeichnet wird, die eine Antwort auf vorgegebene Bedingungen des Herzrhythmus und der Aktivität des Patienten darstellt.

4. Vorrichtung nach Anspruch 1, wobei der Modellzyklus wenigstens einen charakteristischen Verlauf des Minutenvolumens gegenüber dem Herzrhythmus umfasst, der während einer Episode aufgezeichnet wird, die eine Anstrengungsphase, gefolgt von einer Rekonvaleszenzphase des Patienten enthält.

5. Vorrichtung nach Anspruch 1, die außerdem Mittel zum Aktualisieren der Modellzyklen, die von den Mitteln zum Entwickeln und Speichern von Modellzyklen erzeugt werden, umfasst.

6. Vorrichtung nach Anspruch 5, wobei jeder Modellzyklus, der durch die Mittel zum Entwickeln und Speichern von Modellzyklen definiert wird, eine Kombination des letzten erzeugten Modellzyklus mit wenigstens einem früher erzeugten Modellzyklus ist.

7. Vorrichtung nach Anspruch 1, wobei die Analysemittel Mittel sind, die eine Angabe des klinischen Zustands anhand mehrerer gemittelter früherer Modellzyklen berechnen können.

## Claims

1. An active implantable medical device, in particular a stimulation, resynchronisation and/or defibrillation device, or an implant for diagnosis purpose, comprising means for diagnosing cardiac insufficiency, with:
- means (12) for collecting cardiac activity data;
- analysis means adapted to periodically produce at least one index of clinical status of the patient; and
- means (22) for generating a preventive alert for cardiac decompensation as a function of said at least one index of clinical status of the patient;
- means (14) for collecting episodes of reference, these episodes comprising: signals of electric activity of the myocardium; signal of hemodynamic activity of the myocardium; and/or indicators reflecting the variation of physical, activity and/or hemodynamic parameters between phases of effort and phases of recovery;
- discriminator means (10), adapted to analyse said episodes as a function of predefined criteria, so as to assign to each of these episodes a marker of suspicion or non-suspicion of cardiac decompensation; and
- means (16, 18) for elaborating and memorizing modelcycles, adapted to produce based on said episodes at least two model-cycles based on episodes collected in respective situations of suspicion and non-suspicion of cardiac decomposition as determined by the discriminator means (10, 12),
the device being **characterized in that** it further comprises:
- means (20) for characterizing the difference between a suspicion model-cycle and a non-suspicion model-cycle, to produce in response said at least one index of clinical status of the patient.

2. The device of claim 1, wherein said model-cycle comprises at least one beat of endocavitary electrogram recorded during an episode responding to predetermined conditions of cardiac rhythm and activity of the patient.

3. The device of claim 1, wherein said model-cycle comprises at least one beat of endocardial acceleration signal recorded during an episode responding to predetermined conditions of cardiac rhythm and activity of the patient.

4. The device of claim 1, wherein said model-cycle comprises at least one characteristic diagram minuteventilation vs. cardiac rhythm recorded during an episode comprising a phase of effort followed with a phase of recovery of the patient.

5. The device of claim 1, further comprising means for updating the model-cycles produced by said means for elaborating and memorizing model-cycles.

6. The device of claim 5, wherein each model-cycle defined by said means for elaborating and memorizing model-cycles is a combination of the last model-cycle produced with at least one previously produced model-cycle.

7. The device of claim 1, wherein the analysis means are means adapted to calculate a clinical-status index based on a plurality of prior model-cycles averaged together.
